(19) **Europäisches Patentamt**
**European Patent Office**
**Office européen des brevets**

(11) **EP 3 100 678 A1**

(12) **EUROPEAN PATENT APPLICATION**
published in accordance with Art. 153(4) EPC

(43) Date of publication:
**07.12.2016 Bulletin 2016/49**

(51) Int Cl.:
***A61B 5/0408*** (2006.01)     ***A61B 5/0478*** (2006.01)

(21) Application number: **15743324.4**

(86) International application number:
**PCT/JP2015/052226**

(22) Date of filing: **27.01.2015**

(87) International publication number:
**WO 2015/115440 (06.08.2015 Gazette 2015/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.01.2014 JP 2014013789**

(71) Applicants:
• **Nippon Telegraph and Telephone Corporation**
**Tokyo 100-8116 (JP)**
• **Toray Industries, Inc.**
**Tokyo, 103-8666 (JP)**

(72) Inventors:
• **TSUKADA, Shingo**
**Musashino-shi**
**Tokyo 180-8585 (JP)**
• **KASAI, Nahoko**
**Musashino-shi**
**Tokyo 180-8585 (JP)**
• **SUMITOMO, Koji**
**Musashino-shi**
**Tokyo 180-8585 (JP)**
• **TAKAGAHARA, Kazuhiko**
**Musashino-shi**
**Tokyo 180-8585 (JP)**

• **ONO, Kazuyoshi**
**Musashino-shi**
**Tokyo 180-8585 (JP)**
• **KAWANO, Ryusuke**
**Musashino-shi**
**Tokyo 180-8585 (JP)**
• **ISHIHARA, Takako**
**Musashino-shi**
**Tokyo 180-8585 (JP)**
• **KOIZUMI, Hiroshi**
**Musashino-shi**
**Tokyo 180-8585 (JP)**
• **TAKEDA, Keiji**
**Otsu-shi**
**Shiga 520-2141 (JP)**
• **NAGAI, Noriko**
**Otsu-shi**
**Shiga 520-2141 (JP)**
• **ODA, Naoki**
**Tokyo 103-8666 (JP)**
• **TESHIGAWARA, Takashi**
**Tokyo 103-8666 (JP)**

(74) Representative: **Webster, Jeremy Mark et al**
**Mewburn Ellis LLP**
**City Tower**
**40 Basinghall Street**
**London EC2V 5DE (GB)**

(54) **ELECTRODE MEMBER AND DEVICE**

(57)     Provided are an electrode material and a device that can retain a high conductivity after repeated washing and is applicable to bio-electrodes. In a fiber structure constituted of a woven or knitted fabric or the like containing a conductive polymer in the electrode material of the present invention, the conductive polymer such as a polythiophene-based conductive polymer is supported on the surfaces of filaments constituting multifilament that is used in the fiber structure and/or in the gap between the filaments.

Figure 1

EP 3 100 678 A1

**Description**

Field

The present invention relates to an electrode material containing a fiber structure and a conductive polymer, and a device using the electrode material. Specifically, the present invention relates to a textile electrode material that can retain a high conductivity after repeated washing and is applicable to bio-electrodes. Background

Conventionally, materials containing highly conductive metals have been commonly used as electrode materials in view of required properties. Various properties of the shapes of electrodes are also becoming desired along with diversification of uses. To obtain flexible structures that follow and fit various and complex shapes, flexible electrode base materials have been known that can follow the shapes of base materials on which electrodes are disposed or attached. The flexible electrode base materials are produced in the form of a thin metal layer deposited on a film, or by forming the metal itself into a fiber to enhance flexibility, for example.

On the other hand, conductive polymers are attracting attention as a substance having both conductivity of metals and flexibility of organic polymers. Flexible electrodes in which conductive polymers are combined with fiber structures are developed as electrodes alternative to metal electrodes.

In addition, flexible forms are used in recent years in bio-electrodes for acquiring biosignals of living things to follow objects on which the electrodes are attached. Electrodes using hydrogels are commonly used because electrodes of metal materials are partly poor in biocompatibility. However, such electrodes are generally poor in breathability and cause swelling, skin rashes, and the like of the living bodies when being closely attached for a long time, and there has been a strong demand for electrodes that are comfortable to wear.

Electrodes in the form of textiles having conductivity are thought to be particularly effective, and have been developed. For example, it has been developed that textile electrodes are combined with conductive materials impermeable to water to suppress water evaporation from the textile electrodes so that the conductivity is improved (see Patent Literature 1).

It has also been developed that conductive polymer fibers produced by covering part or the whole of conductive polymers such as PEDOT/PSS with thermoplastic resins are applied to sensing materials (see Patent Literature 2 or 3).

However, these developed products have failed to fully utilize the property of being aggregates of filaments, which is an advantage of textiles, and thus failed to provide sufficient electrodes in the form of textiles.

In addition, nanofibers are attracting attention as functional materials in fiber materials, and applications have been developed utilizing their properties. For example, it has been developed that gaps between nanofiber filaments are configured to support functional agents to impart different functionalities (see Patent Literature 4).

Also having been developed regarding electrodes in which nanofibers are used on part of base materials is a technique of conductive compositions exhibiting high conductivities in spite of low conductive polymer contents in terms of the relation between hydrophobic cellulose nanofibers and the conductive polymers (PDOT/PSS), in which the nanofibers are defibrated and the transparency is enhanced to the level that the transparency can be exhibited (see Patent Literature 5).

These documents disclose utilization of nanofibers. The former discloses the functional agents in the gaps between filaments, but there is a problem in obtaining sufficiently practical use because alloy fibers, which form aggregates of extremely short fibers, are used. The latter uses nanofibers, but the developed constitution fails to fully utilize properties of the gaps between filaments and has been poor in practical durability such as washing durability as textile electrodes.

Citation List

Patent Literature

Patent Literature 1: Japanese Patent No. 4860155
Patent Literature 2: Japanese Patent No. 5135757
Patent Literature 3: Japanese Patent Application Laid-open No. 2007-291562
Patent Literature 4: Japanese Patent No. 4581467
Patent Literature 5: Japanese Laid-open Patent Publication No. 2013-216766

Summary

Technical Problem

In view of the above, the present invention has an object to provide an electrode material and a device that can retain a high conductivity after repeated washing and is applicable to bio-electrodes in order to create a practical electrode using a textile base material. Solution to Problem

To solve the above-described problem and achieve the above-described object, an electrode material according to the present invention includes a fiber structure containing a conductive polymer. The conductive polymer is supported on surfaces of filaments constituting the fiber structure and/or in a gap between the filaments.

In the electrode material according to the present invention, the fiber structure includes at least a multifilament yarn, and a conductive substance is supported on surfaces of filaments constituting the multifilament yarn and/or in a gap between the filaments.

In the electrode material according to the present invention, a multifilament yarn constituting the fiber structure includes a filament of equal to or less than 0.2 dtex.

In the electrode material according to the present invention, the conductive polymer is supported on the surfaces of the filaments constituting the fiber structure and/or in the gap between the filaments when the conductive polymer is dispersed with a binder in a solvent and the dispersion in which the conductive polymer is dispersed is applied to the fiber structure.

In the electrode material according to the present invention, the conductive polymer is a mixture of poly(3,4-ethylenedioxythiophene) and polystyrenesulfonic acid.

The electrode material according to the present invention further includes a resin layer layered on one face of the fiber structure containing the conductive substance.

In the electrode material according to the present invention, the electrode material has a surface resistance of equal to or less than $1 \times 10^6$ $\Omega$ after 20 washing cycles in accordance with JIS L0217 (2012) 103 method.

In the electrode material according to the present invention, the electrode material is layered in combination with an adhesive agent.

A device according to the present invention includes the above-described electrode material, the electrode material being used as at least part of an electrode.

Advantageous Effects of Invention

With the present invention, a textile-based electrode material having a high level of conductivity superior in the texture and the washing durability can be obtained and can be preferably used as an electrode of a wearable sensing material, particularly for a use as an electrode for performing sensing of biosignals, that has been difficult to develop with conventional electrodes. Brief Description of Drawings

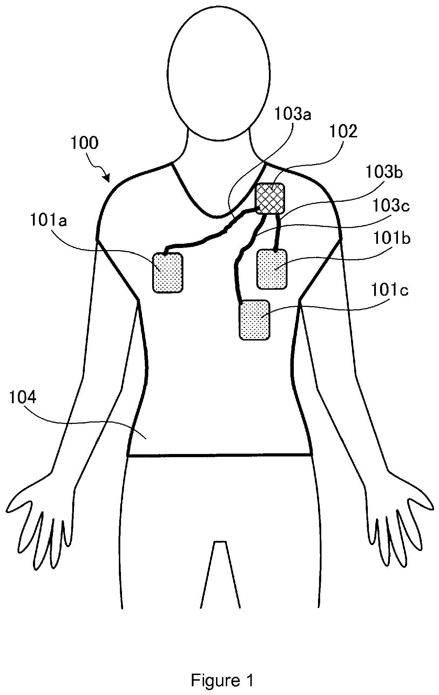

FIG. 1 is a schematic diagram of a biosignal detecting garment in which an electrode material according to an embodiment of the present invention is used. Description of Embodiments

An electrode material according to the present invention will be described below in detail. The embodiment does not limit the present invention.

A preferable aspect of the electrode material of the present invention includes a fiber structure containing a conductive polymer, in which the conductive polymer is supported on the surface of filaments constituting the fiber structure and/or in the gap between the filaments. The conductive polymer here is not limited to particular materials as long as the polymer is a conductive resin. Conductive resin pastes in which carbon black, carbon nanotubes (CNTs), metal nanoparticles, or other substances are contained in resins with low conductivities, and conductive polymers in which the resins themselves have conductivity are preferably used.

The conductive polymer is not limited to particular materials as long as the polymer is conductive. Examples of the conductive polymer include acetylene-based conductive polymers; 5-membered heterocycle-based conductive polymers such as pyrrole-based polymers including polypyrroles, poly(3-alkylpyrrole)s such as a poly(3-methylpyrrole), a poly(3-ethylpyrrole), and a poly(3-dodecylpyrrole), poly(3,4-dialkylpyrrole)s such as a poly(3,4-dimethylpyrrole) and a poly(3-methyl-4-dodecylpyrrole), poly(N-alkylpyrrole)s such as a poly(N-methylpyrrole) and a poly(N-dodecylpyrrole), poly(N-alkyl-3-alkylpyrrole)s such as a poly(N-methyl-3-methylpyrrole) and a poly(N-ethyl-3-dodecylpyrrole), and poly(3-carboxypyrrole)s; thiophene-based polymers including polythiophenes, poly(3-alkylthiophene)s such as a poly(3-methylthiophene), a poly(3-ethylthiophene), and a poly(3-dodecylthiophene), poly(3,4-dialkylthiophene)s such as a poly(3,4-dimethylthiophene) and a poly(3-methyl-4-dodecylthiophene), poly(3-alkoxythiophene)s such as a poly(3-hydroxythiophene) and a poly(3-methoxythiophene), poly(3,4-dialkylthiophene)s such as a poly(3,4-dimethylthiophene) and poly(3,4-dibutylthiophene), poly(3-carboxythiophene)s, poly(3-halothiophene)s such as a poly(3-bromothiophene) and a poly(3-chlorothiophene), and poly(3,4-ethylenedioxythiophene)s; and isothianaphthene-based polymers; aniline-based conductive polymers such as a polyaniline, a poly(2-methylaniline), and a poly(3-isobutylaniline); and phenylene-based conductive polymers such as poly(p-phenylenevinylene)s (PPVs), and copolymers of these polymers. Use of a dopant in combination improves the conductivity of the conductive polymer. The dopant used in combination with the conductive polymer is at least one kind of ions including halide ions such as chloride ions and bromide ions; perchlorate ions; tetrafluoroborate ions; hexafluoroarsenate ions; sulfate ions; nitrate ions; thiocyanate ions; hexafluorosilicate ions; phosphate-based ions such as phosphate ions, phenylphosphate ions, and hexafluorophosphate ions; trifluoroacetate ions; tosylate ions; alkylbenzenesulfonate ions such as ethylbenzenesulfonate ions and dodecylbenzenesulfonate ions; alkylsulfonate ions such as methylsulfonate ions and ethylsulfonate ions; and polymer ions such as polyacrylate ions, polyvinylsulfonate ions, polystyrenesulfonate ions, and poly(2-acrylamido-2-methylpropanesulfonate) ions. The amount of the dopant to be added is not limited to particular values as long as the quantity is sufficient to affect the conductivity.

As the conductive polymer, among the above polymers, a polypyrrole, a poly(3,4-ethylenedioxythiophene) (PEDOT), a polyaniline, a poly(p-phenylenevinylene) (PPV) and the like are easy to resinify and are preferably used in the form of conductive resins. PEDOT/PSS, which is produced by doping PEDOT, a thiophene-based conductive polymer, with a poly(styrenesulfonic acid) (poly(4-styrenesulfonate): PSS), is particularly preferable in terms of safety and workability. In terms of enhancing the conductivity and stabilizing, adding glycerol, a physiological saline solution, or other substances to the fiber structure containing the conductive polymer can be preferably used.

In addition, the fiber structure is preferably impregnated with the conductive polymer such as PEDOT/PSS by applying a dispersion in which the polymer and a binder is dispersed in a solvent to the fiber structure. Using a binder can cause the conductive polymer to be easily supported on the fiber structure and can prevent the surface resistance from rising after repeated washing of the electrode material.

The binder used may be a thermosetting resin or may be a thermoplastic resin. Examples include polyesters such as a polyethylene terephthalate, a polybutylene terephthalate, and a polyethylene naphthalate; polyimides; polyamideimides; polyamides such as a polyamide 6, a polyamide 6,6, a polyamide 12, and a polyamide 11; fluororesins such as a polyvinylidene fluoride, a polyvinyl fluoride, a polytetrafluoroethylene, an ethylene/tetrafluoroethylene copolymer, and a polychlorotrifluoroethylene; vinyl resins such as a polyvinyl alcohol, a polyvinyl ether, a polyvinyl butyral, a polyvinyl acetate, and a polyvinyl chloride; epoxy resins; xylene resins; aramid resins; polyimide silicones; polyurethanes; polyureas; melamine resins; phenolic resins; polyethers; acrylic resins; and copolymers of these polymers. These binders may be dissolved in an organic solvent, may be functionalized with groups such as sulfonate group or carboxy group to form an aqueous solution, or may be dispersed in water by emulsification, for example.

Among the binder resins, preferable resins are at least one of polyurethanes, polyesters, acrylic resins, polyamides, polyimides, epoxy resins, and polyimide silicones because these polymers can be easily mixed.

The solvent used is not limited as long as the conductive polymer and the binder can be stably dispersed, and water or a mixed solution of water and an alcohol can be preferably used. When a polythiophene-based conductive polymer such as PEDOT/PSS is used, a mixed solvent of water and ethanol is preferable.

In terms of enhancing the conductivity and stabilizing of the electrode material, products obtained by further adding glycerol, a physiological saline solution, or other substances to the fiber structure containing the conductive polymer can be preferably used, but the products are not limiting. The conductive polymer can be supported on the surface of filaments constituting the fiber structure and/or in the gap between the filaments by applying precursors of these exemplified conductive polymers, or a solution, an emulsion, a dispersion or the like of the conductive polymers to the fiber structure using a known method such as an immersing method, a coating method, and a spraying method.

The form of the fiber constituting the fiber structure in the electrode material of the present invention may be any of monofilament yarn and multifilament yarn. The cross-sectional shape of the fiber may be a round or triangular cross-section. Other modified cross-sectional shapes with high modification ratios are not particularly limited.

A polymer used as a material for the fiber constituting the fiber structure is not limited to particular polymers as long as the polymer can be formed into a fiber by a known method. The polymer refers to, but is not limited to, polyolefin-based fibers containing a major component such as polyethylene and polypropylene, cellulose for chemical fibers such as rayon and acetate fibers, and polymers for synthetic fibers such as polyesters and nylons.

In the electrode material of the present invention, the fiber constituting the fiber structure preferably has a high and uniform fineness. Preferable examples include thermoplastic polymers, which can be conjugate-spun in melt spinning, particularly fibers made of polyesters.

Examples of the polyesters here include polyesters containing terephthalic acid as a major acid component and containing at least one kind of glycols selected from $C_{2-6}$ alkylene glycols, that is, ethylene glycol, trimethylene glycol, tetramethylene glycol, pentamethylene glycol, and hexamethylene glycol, preferably selected from ethylene glycol and tetramethylene glycol, particularly preferably containing ethylene glycol as a major glycol component.

The polyesters may be polyesters in which the acid component is a mixture of terephthalic acid and another bifunctional carboxylic acid, or may be polyesters in which the glycol component is a mixture of the above glycol and another diol component. In addition, the polyesters may be polyesters in which the acid component is a mixture of terephthalic acid and another bifunctional carboxylic acid and the glycol component is a mixture of the above glycol and another diol component.

Examples of the bifunctional carboxylic acid other than terephthalic acid used here include aromatic, aliphatic, and alicyclic bifunctional carboxylic acids such as isophthalic acid, naphthalenedicarboxylic acids, diphenyldicarboxylic acids, diphenoxyethanedicarboxylic acids, adipic acid, sebacic acid, and 1,4-cyclohexanedicarboxylic acid. Examples of the diol compound other than the above glycols include aromatic, aliphatic, and alicyclic diol compounds such as cyclohexane-1,4-dimethanol, neopentyl glycol, bisphenol A, and bisphenol S.

The polyesters used as the fiber constituting the fiber structure may be synthesized by any method. For example, a polyethylene terephthalate can be commonly produced by a first-stage reaction that generates a glycol ester of terephthalic acid and/or its low polymer by a direct esterification reaction of terephthalic acid with ethylene glycol, a transesterification reaction of a lower alkyl ester of terephthalic acid such as dimethyl terephthalate with ethylene glycol, or a reaction of terephthalic acid with ethylene oxide, and a second-stage reaction in which the first-stage reaction product is heated under reduced pressure to cause a polycondensation reaction until a desired degree of polymerization is obtained.

The form of the fiber structure according to the present invention may be any forms appropriate to the intended use such as mesh, paper, woven fabric, knitted fabric, nonwoven fabric, ribbon, and string and is not limited to particular forms.

When the electrode material of the present invention is used as a bio-electrode, the form of the fiber structure is preferably the form of woven fabric, knitted fabric, or nonwoven fabric in terms of adhesion and followability to the skin surface and flexible and soft textures and because a high breathability is demanded to prevent stuffy feelings and skin rashes due to sweat on the skin surface.

Performing dyeing, treatments to impart functions, and the like by known methods or means on these fiber structures is not limited as long as performances as an electrode is not impaired. Also, performing physical surface treatments such as nap raising, calendering, embossing, and waterjet punching on the surface of the electrode material is not limited as long as performances as an electrode is not impaired.

In a preferable aspect of the present invention, the fiber structure includes at least multifilament yarn, and a conductive substance is supported on the surface of filaments constituting the multifilament yarn and/or in the gap between the filaments.

In terms of supporting of the conductive polymer on the fiber structure and high conductivity of the electrode material, the fiber structure preferably contains multifilament yarn constituted by a plurality of filaments. The fineness of the multifilament yarn is not limited to particular values and is preferably 30 dtex to 400 dtex in terms of utilizing properties as a fiber structure. The mixing ratio of the multifilament yarn in the fiber structure is not limited to particular values to the extent that the performances are not affected. The mixing ratio is preferably high in terms of easy supporting of the conductive resin and enhancing practical durability. The multifilament yarn used can be twisted, doubled, or crimped by known methods.

In a more preferable aspect, the multifilament contained in the fiber structure contains filaments of equal to or less than 0.2 dtex. In terms of supporting of the conductive polymer on the fiber structure and high conductivity, it is desirable that the fiber structure contains filaments of a small fiber diameter, and filaments of equal to or less than 0.2 dtex are preferably contained. In an example of polyethylene terephthalate having a density of 1.38 g/cm$^3$, a fineness of 0.2 dtex results in a microfiber having a fiber diameter of about 5 $\mu$m. A microfiber of equal to or less than 0.2 dtex made of a polymer compound having a density that allows forming the compound into a fiber is a fiber of a sufficiently high fineness and can form a large number of gaps by the filaments.

As the number of the filaments constituting the multifilament increases, the gaps formed by the filaments, in other words, sites on which the conductive polymer is supported, are redifferentiated, and the conductive polymer is well supported on the fiber structure. In addition, even when the sites capable of supporting the conductive polymer are redifferentiated because of the smaller fiber diameters of the filaments, continuity of the conductive polymer is retained, and a high conductivity can also be exhibited at the same time.

For example, as a microfiber with a large number of filaments, sea-island composite fiber yarn containing two polymers having different solubilities is prepared, and one nature of the sea-island composite fiber is removed with a solvent to form the yarn into an ultrafine fiber. The diameter and the distribution of each island component are not fixed. Multifilament made of a microfiber can be formed by increasing the number of filaments constituting the island component.

In the multifilament produced by the above method, the number of filaments constituting the island component of the microfiber is equal to or more than 5, preferably equal to or more than 24, and more preferably equal to or more than 50, although the number depends on the filament fineness and whether the filaments are twisted, for example. In addition, the present invention also includes denier-mixed fibers. The overall cross-sectional form of the multicomponent fiber is not limited to round holes and includes forms of every publicly known fiber cross-sections such as trilobal types, tetralobal types, T-types, and hollow types.

In a preferable mode of the fiber structure according to the present invention, one of preferable aspects is produced by treating woven fabric woven with a sea-island composite fiber by a method such as chemical peeling, physical peeling, and dissolution removal to produce woven or knitted fabric in which the constituent fiber has been formed into an ultrafine fiber, and entangling the fiber filaments with each other by waterjet punching, for example.

In the above preferable mode of the fiber structure, an elastic polymer substance such as a polyurethane is added by means such as impregnation in order to retain the entangled structure of the fiber. This has effects of improving dyeability, dimensional stability, qualitative stability, and other properties of the fiber structural part object. Furthermore, various types of sheet-shaped products appropriate to the purpose can be obtained by raising a nap on the surface of a sheet-shaped fiber structure to form raised bundles of the ultrafine fiber on the surface, for example.

The fiber structure is subjected to a large number of treatments such as shrinkage treatment, form-fixing treatment, compression treatment, dyeing and finishing treatment, oil-adding treatment, heat-fixing treatment, solvent removal, removal of form-fixing agents, combing treatment, calendering treatment, flat (roll) breath treatment, and high-performance short-cut shirring treatment (cutting raised fibers) in addition to entangling and nap raising of the fiber performed at corresponding steps of corresponding processes in combination as appropriate, but the performance of the treatments is not limited as long as performances as an electrode is not impaired.

Furthermore, in the fiber structure according to the present invention, the filaments constituting the multifilament are more preferably a nanofiber having a fiber diameter of 0.01 dtex to 0.0001 dtex inclusive, and fiber structures containing multifilament thread constituted of nanofibers produced by known methods such as aggregates of nanofiber staple yarn made of "nanoalloy (registered trademark)" fibers and aggregates of monofilament yarn made by an electrospinning method or other methods can be preferably used.

The multifilament yarn constituted of a nanofiber can be produced by a known conjugate-spinning method, for example. An example that can be effectively used is nanofiber multifilament yarn having small fiber diameter variations obtained by removing the sea components from composite fibers obtained using composite spinnerets exemplified in Japanese Patent No. 5472479 and Japanese Patent Application Laid-open No. 2013-185283 (Fibers & Textiles Research Laboratories VESTA patents), but this example is not limiting.

The cross-sectional shape of the filaments is also not limited to particular shapes, and the shape may be a publicly known cross-sectional shape such as round, triangular, flat, and hollow shapes. Multifilament yarns having a diversity of cross-sectional forms of fibers obtained using the composite spinnerets exemplified in Japanese Patent Application Laid-open No. 2013-185283, particularly having cross-sections of high modification ratios (in the modification ratio in the present invention, the modification ratio is higher when the ratio of the circumscribed circle to the inscribed circle of yarns having different diameters (circumscribed circle/the inscribed circle) is larger) can be preferably used.

The thickness of the fiber structure used for the electrode material of the present invention is preferably equal to or more than 0.2 mm and equal to or less than 2.0 mm. When the thickness is less than 0.2 mm, the substantial areal weight is small due to the too small thickness of the cloth, and the quantity of the conductive polymer impregnated is small. A thickness of larger than 2.0 mm may cause uncomfortable wearing due to the too large thickness. Equal to or more than 0.3 mm and equal to or less than 1.5 mm is more preferable. The size of the electrode material is not particularly specified as long as signals can be detected, and each of the length and the breadth is preferably equal to or more than 2 cm and equal to or less than 20 cm. A length or a breadth of equal to or less than 2 cm leads to a too small area of the electrode material, which results in a higher possibility of sliding of the electrode during actions or exercise and a resulting higher possibility of picking up noise. A length or a breadth of equal to or more than 20 cm is larger than the size substantially required for detecting signals and may cause uncomfortable wearing due to the too large area of the electrode material. Each of the length and the breadth is more preferably equal to or more than 2.5 cm and equal to or less than 18 cm.

In the electrode material of the present invention, a resin layer is preferably layered on one face of the fiber structure containing the conductive polymer.

In particular, in consideration of application of the electrode material to bio-electrodes, the resin layer is preferably formed on the face of the electrode material opposite to the face configured to have contact with the skin surface of a human body. The electrode material including the resin layer enables control of the humidity of an electrode material portion, which enables stable conductivity to be exhibited. Covering one face of the electrode material with the resin layer can considerably prevent impairing of durability of the electrode material, particularly impairing of the conductivity due to falling off of the conductive polymer caused by washing. The kind and the shape of the polymer constituting the resin layer are not limited as long as humidity control is enabled, and a waterproof moisture-permeable layer having an insulating property is preferable in view of desired properties as an electrode material.

Examples of the waterproof moisture-permeable layer include, but are not limited to, forms obtained by layering known membranes, films, laminates, resins, and the like such as polytetrafluoroethylene (PTFE) porous membranes, non-porous membranes of hydrophilic elastomers such as hydrophilic polyester resins and polyurethane resins, and polyurethane-resin microporous membranes by a coating or lamination method, in terms of discharging vapor sweat. The waterproof moisture-permeable layer is preferably a layer obtained by laminate-bonding an elastic polyurethane-resin microporous membrane by laminating in terms of followability to the fiber structure, which is the base material.

The electrode material of the present invention preferably has a surface resistance of equal to or less than 1

x $10^6$ $\Omega$ after 20 washing cycles in accordance with JIS L-0217 (2012) 103 method. The electrode material of the present invention contains the fiber structure and the conductive polymer and can be home laundered. As the number of the filaments constituting the fiber structure increases, the gaps formed by the filaments, in other words, sites on which the conductive polymer is supported, are redifferentiated, and the conductive polymer is well supported on the fiber structure. Thus, it is thought that a high washing durability can be imparted.

Examples of preferable aspects of use of the electrode material of the present invention include adhesive electrodes, in which an adhesive agent is combined utilizing the properties as a textile electrode, and devices in which the electrode material of the present invention is used as at least part of electrodes.

A first example of devices using the electrode material of the present invention is various sensing apparatuses, and stationary types, mobile types, wearable types, and other types are exemplified. As sensing use, the present invention is applicable to measurements of heart rates, cardiographic waveforms, respiratory rates, blood pressures, brain potentials, myogenic potentials, and the like, which are sensing use obtained from electric signals from living bodies. Examples include, but are not limited to, daily health management, health management during leisure activities and exercise, and remote management of heart disease, high blood pressure, the sleep apnea syndrome. In addition to sensing use, examples include low-frequency massagers and muscle-stimulation muscle-strengthening devices as devices for sending electricity to bodies.

FIG. 1 is a schematic diagram of a biosignal detecting garment 100 in which the electrode material of the present invention is used. Two of electrode materials 101 (101a, 101b, and 101c) according to the present invention are placed on portions of a garment body 104 that are configured to have contact with about right and left sides of the chest or the flank when the garment is worn, and the remaining one is placed at a lower position separated from the electrode materials placed about right and left sides of the chest or the flank of the garment body 104. Each electrode material 101 measures biosignals. The biosignals measured by the electrode materials 101 are transmitted to a measurement device 102 via wires 103 (103a, 103b, and 103c). The biosignals transmitted to the measurement device 102 are subjected to signal processing and then transmitted to a mobile terminal or a personal computer. The electrode materials 101 of the present invention can stably detect biosignals when used as wearable electrodes such as the biosignal detecting garment 100 illustrated in FIG. 1.

Examples

Next, the electrode material of the present invention will be described in detail with reference to examples. The electrode material of the present invention is not limited to these examples. Measured values in the examples and comparative examples were obtained by the following methods.

(1) Fineness

For sea-island composite fibers, a fabric was immersed in a 3% by mass aqueous solution of sodium hydroxide (75°C, with a bath ratio of 1:30) to dissolve and remove equal to or more than 99% of easily soluble components. Threads were then disassembled to select a multifilament constituted of ultrafine fiber filaments, and the mass of 1 m of the multifilament was measured. The fineness was calculated by multiplying the mass by 10,000. The procedure was repeated 10 times, and the fineness was defined as the value obtained by rounding the simple average to the first decimal place.

For other fibers, threads were disassembled to select a multifilament, and the mass of 1 m of the multifilament was measured. The fineness was calculated by multiplying the mass by 10,000. The procedure was repeated 10 times, and the fineness was defined as the value obtained by rounding the simple average to the first decimal place.

(2) Fiber Diameter

The obtained multifilament was embedded in an epoxy resin, frozen with an FC-4E cryosectioning system manufactured by Reichert, Inc., and cut with Reichert-Nissei Ultracut N (an ultramicrotome) equipped with a diamond knife. The cut surface was photographed with a VE-7800 scanning electron microscope (SEM) manufactured by KEYENCE Corp. at a magnification of 5,000 for nanofibers, 1,000 for microfibers, and 500 for the others. From the photographs obtained, 150 ultrafine fiber filaments randomly selected were sampled, and every circumscribed circle diameter (fiber diameter) was measured using image processing software (WINROOF) on the photographs.

(3) Fiber Diameter and Variation of Fiber Diameter (CV% (A)) of Multifilament

The average fiber diameter and the standard deviation of the fiber diameters of the above fiber diameters were calculated, and a fiber diameter CV% (coefficient of variation) was calculated on the basis of the equation below. All the above values are obtained by performing measurements for each photograph of 3 sites, obtaining the average value of the 3 sites, performing measurements in units of nanometers to one decimal place, and rounding the values to the whole number.

$$\text{The variation of fiber diameter (CV\% (A))} = \text{(the standard deviation of fiber diameters/the average fiber diameter)} \times 100$$

(4) Modification Ratio and Variation of Modification Ratios (CV% (B))

By a method similar to the above method for fiber diameters, the cross-section of the multifilament was photographed, and a circumscribed circle diameter (the fiber diameter) was defined as the diameter of a perfect circle circumscribing the cut plane on the basis of the image. In addition, an inscribed circle diameter was defined as the diameter of an inscribed perfect circle, and a value was calculated to three decimal places by the modification ratio = the circumscribed circle diameter ÷ the inscribed circle diameter. The value was rounded to two decimal places to obtain the modification ratio. The modification ratios were measured for 150 ultrafine fiber filaments randomly sampled in the same image, and a variation of modification ratios (CV% (B) (coefficient of variation)) was calculated from the average value and the standard deviation on the basis of the equation below.
This variation of modification ratios is rounded to one decimal place.

$$\text{The variation of modification ratios (CV\% (B))} = \text{(the standard deviation of modification ratios/the average value of modification ratios)} \times 100 \ (\%)$$

(5) Quantity of Attached Resin

A quantity of the attached resin was measured on the basis of changes in the mass of a fiber structure that was a test fabric between before and after applying a dispersion of a conductive polymer in the standard state (20°C x 65% RH). The calculation expression is as follows.
The quantity of the attached resin $(g/m^2)$ (the mass of the test fabric after being treated (g) - the mass or the weight of the test before being treated (g))/the area of the test fabric on which the dispersion is applied $(m^2)$

(6) Surface Resistance

An electrode of 10 cm x 10 cm was used as a test piece and placed on high-quality expanded polystyrene. A surface resistance value ($\Omega$) was measured with a resistance meter (Loresta-AX MCP-T370, a 4-probe resistance meter manufactured by Mitsubishi Chemical Analytech Co., Ltd.) under an environment of 20°C and 40% RH.

(7) Washing Durability

An electrode of 10 cm x 10 cm was used as a test piece, and a surface resistance was measured after washing by a 20-time repeating method by a method in accordance with JIS L0217 (2012) 103 method. An automatic washing machine (National NA-F50Z8) was used as the washing machine.

(8) Breathability

The breathability of an electrode was measured in accordance with the air permeability A method (the Frazier method) in JIS L 1096 (testing methods for woven and knitted fabrics) (1999).

(9) Bending Resistance

The bending resistance of the electrode was measured in accordance with the bending resistance A method (the 45° cantilever method) in JIS L 1096 (testing methods for woven and knitted fabrics) (1999).
Examples of the electrode material according to the present invention will be described.

[Example 1]

A tubular knitted fabric was knitted into an interlock structure using a polyester-nanofiber combined-filament yarn of 100T-136F obtained by combining a high-shrinkage yarn of 22T-24F with a nanofiber of 75T-112F (with a composite ratio of the sea/island components of 30%:70%, the number of the islands of 127/F) of an alkaline-hot-water soluble polyester in which the island component was polyethylene terephthalate and the sea component was a polyester copolymer containing terephthalic acid and sodium 5-sulfoisophthalate as the acid component. Next, the fabric was immersed in a 3% by mass aqueous solution of sodium hydroxide (75°C, with a bath ratio of 1:30) to remove easily soluble components. A knitted fabric was obtained using the combined-filament yarn of the nanofiber and the high-shrinkage yarn. To the knitted fabric obtained as a fiber structure, a dispersion in which 1.0% by weight of PEDOT/PSS as a conductive polymer and 5.0% by weight of an acrylic thermosetting resin as a binder were dispersed in a mixed solvent of water and ethanol (44% by weight of water and 50% by weight of ethanol) was applied by a known gravure coating method so that the quantity of the applied agent would be 15 g/m$^2$ to obtain an electrode. Table 1 and Table 2 list the materials used and properties of the obtained electrode.

[Example 2]

An electrode was produced through the same treatments as those for Example 1 except that the high-shrinkage yarn of 22T-24F was changed to a high-shrinkage yarn of 33T-6F and a polyester-nanofiber combined-filament yarn of 110T-118F obtained by crossing the high-shrinkage yarn with the nanofiber of 75T-112F (with a composite ratio of the sea/island components of 30%:70%, the number of the islands of 127/F) was used. Table 1 and Table 2 list the materials used and properties of the obtained electrode.

[Example 3]

An electrode was produced through the same treatments as those for Example 1 except that the fabric structure was changed from knitted fabric to plain weave fabric. Table 1 and Table 2 list the materials used and properties of the obtained electrode.

[Example 4]

An electrode was produced through the same treatments as those for Example 1 except that the high-shrinkage yarn of 22T-24F was not used and the yarn was changed to a polyester-nanofiber single yarn of 75T-112F (with a composite ratio of the sea/island components of 30%:70%, the number of the islands of 127/F). Table 1 and Table 2 list the materials used and properties of the obtained electrode.

[Example 5]

An electrode was produced through the same treatments as those for Example 1 except that the high-shrinkage yarn of 22T-24F was not used and the yarn of 75T-112F (with a composite ratio of the sea/island components of 30%:70%, the number of the islands of 127/F) was changed to a polyester-nanofiber single yarn of 100T-30F (with a composite ratio of the sea/island components of 30%:70%, the number of the islands of 2048/F). Table 1 and Table 2 list the materials used and properties of the obtained electrode.

[Example 6]

An electrode was produced through the same treatments as those for Example 1 except that the high-shrinkage yarn of 22T-24F was not used and the yarn of 75T-112F (with a composite ratio of the sea/island components of 30%:70%, the number of the islands of 127/F) was changed to a polyester-nanofiber single yarn of 120T-60F (with a composite ratio of the sea/island components of 50%:50%, the number of the islands of 2048/F). Table 1 and Table 2 list the materials used and properties of the obtained electrode.

[Example 7]

An electrode was produced through the same treatments as those for Example 1 except that the high-shrinkage yarn of 22T-24F was not used and the yarn was changed to a polyester-nanofiber single yarn of 75T-112F (with a composite ratio of the sea/island components of 30%:70%, the number of the islands of 127/F) having a triangular cross-section. Table 1 and Table 2 list the materials used and properties of the obtained electrode.

[Example 8]

An electrode was produced through the same treatments as those for Example 1 except that the high-shrinkage yarn of 22T-24F was not used and the fabric was changed from a fabric of 75T-112F (with a composite ratio of the sea/island components of 30%:70%, the number of the islands of 127/F) to a woven fabric of a microfiber of 66T-9F (with a composite ratio of the sea/island components of 20%:80%, the number of the islands of 70/F). Table 1 and Table 2 list the materials used and properties of the obtained electrode.

[Example 9]

A polyurethane was added by impregnation to a needle-punched nonwoven fabric having been formed using a polymer array fiber (with a composite ratio of the sea/island components of 57%:43%, the number of the islands of 16) of 4.2 dtex and 51 mm in which the island component was polyethylene terephthalate and the sea component was polystyrene, and wet-solidifying was performed. The content of the polyurethane was 49% of the mass of polyethylene terephthalate. The product was immersed in trichloroethylene and squeezed with a mangle to remove the polystyrene component. An ultrafine fiber having a single-yarn fineness of 0.15 dtex was obtained. A nonwoven fabric having been subjected to nap raising with a buffing machine and dyeing was obtained. In the same manner as in Example 1, a dispersion in which PEDOT/PSS as a conductive polymer and an acrylic thermosetting resin as a binder were dispersed in a mixed solvent of water and ethanol was then applied to the nonwoven fabric obtained as a fiber structure by a known gravure coating method so that the quantity of the applied agent would be 15 g/m$^2$ to obtain an electrode. Table 1 and Table 2 list the materials used and properties of the obtained electrode.

[Example 10]

An electrode was produced through the same treatments as those for Example 1 except that the high-shrinkage yarn of 22T-24F was not used and the fabric was changed from a fabric of 75T-112F (with a composite ratio of the sea/island components, the number of the islands of 127/F) to a woven fabric of a polyester fiber of 84T-36F (a polyester fiber cloth for dyeing tests manufactured by Shikisensha Co., Ltd.). Table 1 and Table 2 list the materials used and properties of the obtained electrode.

[Example 11]

A tubular knitted fabric was knitted using a combined-filament yarn obtained by combining a polyester fiber of 56T-24F with a polyurethane yarn. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Example 1, a dispersion of a conductive polymer was applied to the knitted fabric obtained as a fiber structure to obtain an electrode. Table 1 and Table 2 list the materials used and properties of the obtained electrode.

[Example 12]

A tubular knitted fabric was knitted using nylon-fiber single yarn of 78T-24F. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Example 1, a dispersion of a conductive polymer was applied to the knitted fabric obtained as a fiber structure to obtain an electrode. Table 1 and Table 2 list the materials used and properties of the obtained electrode.

[Example 13]

A tubular knitted fabric was knitted using a polyester-nanofiber combined-filament yarn of 100T-136F obtained by combining a nanofiber of 75T-112F (with a composite ratio of the sea/island components of 30%:70%, the number of the islands of 127/F) with a high-shrinkage yarn of 22T-24F. Next, the fabric was immersed in a 3% by mass aqueous solution of sodium hydroxide (75°C, with a bath ratio of 1:30) to remove easily soluble components. A knitted fabric was obtained using the combined-filament yarn of the nanofiber and the high-shrinkage yarn. A polyurethane-resin microporous membrane was laminated on the back face of the obtained knitted fabric by a known method. A dispersion in which PEDOT/PSS as a conductive polymer and an acrylic thermosetting resin as a binder were dispersed in a mixed solvent of water and ethanol was applied to the front face by a known gravure coating method so that the quantity of the applied agent would be 15 g/m$^2$ to obtain an electrode. Table 1 and Table 2 list the materials used and properties of the obtained electrode.

[Example 14]

An electrode was obtained through the same treatments as those for Example 13 except that the high-shrinkage yarn of 22T-24F was changed to a high-shrinkage yarn of 33T-6F and a polyester-nanofiber combined-filament yarn obtained by combining the high-shrinkage yarn with 75T-112F (with a composite ratio of the sea/island components of 30%:70%, the number of the islands of 127/F) was used. Table 1 and Table 2 list the materials used and properties of the obtained electrode.

[Example 15]

An electrode was obtained through the same treatments as those for Example 13 except that the fabric structure was changed from knitted fabric to plain weave fabric. Table 1 and Table 2 list the materials used and properties of the obtained electrode.

[Example 16]

An electrode was obtained through the same treatments as those for Example 13 except that the yarn was changed to a polyester-nanofiber single yarn of 75T-112F (with a composite ratio of the sea/island components of 30%:70%, the number of the islands of 127/F). Table 1 and Table 2 list the materials used and properties of the obtained electrode.

[Example 17]

An electrode was obtained through the same treatments as those for Example 13 except that the yarn was changed to a polyester-nanofiber single yarn of 100T-30F (with a composite ratio of the sea/island components of 30%:70%, the number of the islands of 2048/F). Table 1 and Table 2 list the materials used and properties of the obtained electrode.

[Example 18]

An electrode was obtained through the same treatments as those for Example 13 except that the yarn was changed to a polyester-nanofiber single yarn of 120T-60F (with a composite ratio of the sea/island components of 50%:50%, the number of the islands of 2048/F). Table 1 and Table 2 list the materials used and properties of the obtained electrode.

[Example 19]

An electrode was obtained through the same treatments as those for Example 13 except that the yarn was changed to a polyester-nanofiber single yarn of 75T-112F (with a composite ratio of the sea/island components of 30%:70%, the number of the islands of 127/F) having a triangular cross-section. Table 1 and Table 2 list the materials used and properties of the obtained electrode.

[Example 20]

An electrode was obtained through the same treatments as those for Example 13 except that the high-shrinkage yarn of 22T-24F was not used and the tubular knitted fabric was changed to a tubular knitted fabric obtained using a microfiber of 66T-9F (with a composite ratio of the sea/island components of 20%:80%, the number of the islands of 70/F). Table 1 and Table 2 list the materials used and properties of the obtained electrode.

[Example 21]

A polyurethane was added by impregnation to a needle-punched nonwoven fabric having been formed using a polymer array fiber (with a composite ratio of the sea/island components of 57%:43%, the number of the islands of 16) of 4.2 dtex and a length of 51 mm in which the island component was polyethylene terephthalate and the sea component was polystyrene, and wet-solidifying was performed. The content of the polyurethane was 49% of the mass of polyethylene terephthalate. The product was immersed in trichloroethylene and squeezed with a mangle to remove the polystyrene component. An ultrafine fiber having a single-yarn fineness of 0.15 dtex was obtained. A nonwoven fabric having been subjected to nap raising with a buffing machine and dyeing was obtained. In the same manner as in Example 13, a polyurethane-resin microporous membrane was laminated on the back face of the obtained nonwoven fabric, and a dispersion of a conductive polymer was applied to the front face to obtain an electrode. Table 1 and Table 2 list the materials used and properties of the obtained electrode.

[Example 22]

Using a polyester-fiber woven fabric of 84T-36F (a polyester fiber cloth for dyeing tests manufactured by Shikisensha Co., Ltd.), a polyurethane-resin microporous membrane was laminated on the back face of a fabric, and a dispersion of a conductive polymer was applied to the front face to obtain an electrode in the same manner as in Example 13. Table 1 and Table 2 list the materials used and properties of the obtained electrode.

[Example 23]

A tubular knitted fabric was knitted using a combined-filament yarn obtained by combining a polyester fiber of 56T-24F with a polyurethane yarn. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Example 13, a polyurethane-resin microporous membrane was laminated on the back face of the obtained knitted fabric, and a dispersion of a conductive polymer was applied to the front face to obtain an electrode. Table 1 and Table 2 list the materials used and properties of the obtained electrode.

[Example 24]

A tubular knitted fabric was knitted using a nylon-fiber single yarn of 78T-24F. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. A polyurethane-resin microporous membrane was laminated on the back face of the obtained knitted fabric, and a dispersion of a conductive polymer was applied to the front face to obtain an electrode. Table 1 and Table 2 list the materials used and properties of the obtained electrode.

[Example 25]

An electrode was obtained through the same treatments as those for Example 1 except that the conductive polymer was changed to a 5% polyaniline aqueous solution (manufactured by Sigma-Aldrich Co. LLC.). Table 1 and Table 2 list the materials used and properties of the obtained electrode.

[Example 26]

An electrode was obtained through the same treatments as those for Example 1 except that the conductive polymer was changed to a 5% polypyrrole aqueous solution (manufactured by Sigma-Aldrich Co. LLC.). Table 1 and Table 2 list the materials used and properties of the obtained electrode.

[Example 27]

An electrode was obtained through the same treatments as those for Example 1 except that the polyester nanofiber of Example 4 was changed to a nylon nanofiber. Table 1 and Table 2 list the materials used and properties of the obtained electrode.

[Example 28]

As an example of an apparatus using the electrode material according to the present invention, 110T-34F of silver-coated thread "AGposs" manufactured by Mitsufuji Textile Ind. Co., Ltd. was caused to pass through a vinyl insulating-system tube and to protrude from one end of the tube. The silver-coated thread protruding from one end was connected by sewing in to the electrode of Example 1 having cut into 3 cm square. On a face on which the silver-coated thread was positioned, a waterproof moisture-permeable surgical sheet "Tegaderm Smooth Film Roll" manufactured by 3M Health Care Limited was attached from above to produce an electrode for electrocardiograms.

[Example 29]

As an example of an apparatus using the electrode according to the present invention, the electrodes described in Example 1 cut into a size of 7 cm by 5 cm were sewed with sewing thread as the different electrodes on right and left sides of the chest inside a commercially available stretch sports inner. In addition, the electrode of Example 1 cut into the same size of 7 cm by 5 cm was sewed with sewing thread as the indifferent electrode (the reference biopotential electrode) at a position 5 cm below the electrode on the left side of the chest. In addition, 110T-34F of the silver-coated thread "AGposs" manufactured by Mitsufuji Textile Ind. Co., Ltd. as wires were sewed with a sewing needle on the inner from each of the three electrode portions to the left clavicular region so that the wires would not have contact with each other. Waterproof seam tape "αE-110" manufactured by Toray Coatex Co., Ltd. was attached on the front and back faces of the wiring portions of the silver-coated thread to insulate and cover the wiring portions. A signal detecting device was attached and connected to the silver-coated thread drawn to the left clavicular region to produce a wearable electrode inner that could measure electrocardiograms when being worn.

[Comparative Example 1]

A conductive polymer PEDOT/PSS (SEP LYGIDA (registered trademark) manufactured by Shin-Etsu Polymer Co., Ltd.) and an acrylic resin were applied to a PET film by a known gravure coating method so that the quantity of the applied agent would be 15 g/m$^2$ in the same manner as in Example 1 to obtain an electrode. Table 1 and Table 2 list the materials used and properties of the obtained electrode.

[Comparative Example 2]

A conductive polymer sticky hydrogel was applied to a PET film by the same known gravure coating method as in Example 1 so that the quantity of the applied resin would be 15 g/m$^2$ to obtain an electrode. Table 1 and Table 2 list the materials used and properties of the obtained electrode.

Table 1

| | Filament | Polymer | Cross-section | Fineness | Fiber diameter | Use of yarn | Variation of fiber diameter (CV% (A)) | Variation of modification ratio (CV% (B)) | Density (yarns /in) length x breadth | Areal weight (g/cm$^2$) | Fiber structure | Conductive polymer | Quantity of applied resin (g/cm$^2$) |
|---|---|---|---|---|---|---|---|---|---|---|---|---|---|
| Example 1 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | PEDOT/PSS | 14.3 |
| Example 2 | Multifilament /High-shrinkage yarn | Polyester | Round | 0.004 dtex /5.5 dtex | 700 nm | 75T-112F (island component 30%:70%) /33T-6F | 5 | 7 | 46 × 110 | 194 | Knitted fabric | PEDOT/PSS | 14.5 |
| Example 3 | Multifilament /High-shrinkage yarn | Polyester | Round | 0.004 dtex /0.9 dtex | 700 nm | 75T-112F (island component 30%:70%) /22T-24F | 5 | 7 | 216 × 113 | 98 | Woven fabric | PEDOT/PSS | 11.2 |
| Example 4 | Multifilament | Polyester | Round | 0.004 dtex | 700 nm | 75T-112F (island component 30%:70%) | 5 | 7 | 43 × 58 | 112 | Knitted fabric | PEDOT/PSS | 13.2 |
| Example 5 | Multifilament | Polyester | Round | 0.001 dtex | 300 nm | 100T-30F (island component 30%:70%) | 3 | 3.4 | 58 × 78 | 110 | Knitted fabric | PEDOT/PSS | 14.8 |
| Example 6 | Multifilament | Polyester | Round | 0.0004 dtex | 200 nm | 120T-60F (island component 50%:50%) | 3 | 3.4 | 70 × 94 | 98 | Knitted fabric | PEDOT/PSS | 15.3 |

| | Filament | Polymer | Cross-section | Fineness | Fiber diameter | Use of yarn | Variation of fiber diameter (CV% (A)) | Variation of modification ratio (CV% (B)) | Density (yarns /in) length x breadth | Areal weight (g/cm$^2$) | Fiber structure | Conductive polymer | Quantity of applied resin (g/cm$^2$) |
|---|---|---|---|---|---|---|---|---|---|---|---|---|---|
| Example 7 | Multifilament | Polyester | triangular | 0.004 dtex | 700 nm | 75T-112F (island component 30%:70%) | 3 | 3.4 | 43 × 58 | 115 | Knitted fabric | PEDOT/PSS | 13.0 |
| Example 8 | Multifilament | Polyester | Round | 0.07 dtex | 2700 nm | 66T-9F (island component 20% : 80%) | 6 | 9 | 114 × 118 | 61 | Woven fabric | PEDOT/PSS | 10.2 |
| Example 9 | Multifilament | Polyester | Round | 0.15 dtex | 3800 nm | 0.15 dtex single-yarn fineness | 6 | 9 | - | 135 | Nonwoven fabric | PEDOT/PSS | 15.2 |
| Example 10 | Multifilament | Polyester | Round | 2.3 dtex. | 15000 nm | 84T-36F | 4 | 4.2 | 105 × 95 | 68 | Woven fabric | PEDOT/PSS | 12.8 |
| Example 11 | Multifilament | Polyester /Polyurethane | Round | 2.3 dtex. | 15000 nm | 56T-24F /22T (PU) | 4 | 4.2 | 67 × 62 | 176 | Knitted fabric | PEDOT/PSS | 13.9 |
| Example 12 | Multifilament | Nylon | Round | 3.3dtex | 36000 nm | 78T-24F | 3.5 | 3.7 | 32 × 40 | 88 | Knitted fabric | PEDOT/PSS | 13.2 |
| Example 13 | Multifilament /high-shrinkage yarn | Polyester | Round | 0.004 dtex /0.9 dtex. | 700 nm | 75T-112F (island component 30%:70%) /22T-24F | 5 | 7 | 58 × 78 | 118 | Knitted fabric | PEDOT/PSS | 15.5 |
| Example 14 | Multifilament /High-shrinkage yarn | Polyester | Round | 0.004 dtex /5.5 dtex | 700 nm | 75T-112F (island component 30%:70%) /33T-6F | 5 | 7 | 46 × 110 | 194 | Knitted fabric | PEDOT/PSS | 15.3 |

(continued)

| | Filament | Polymer | Cross-section | Fineness | Fiber diameter | Use of yarn | Variation of fiber diameter (CV% (A)) | Variation of modification ratio (CV% (B)) | Density (yarns/in) length x breadth | Areal weight (g/cm$^2$) | Fiber structure | Conductive polymer | Quantity of applied resin (g/cm$^2$) |
|---|---|---|---|---|---|---|---|---|---|---|---|---|---|
| Example 15 | Multifilament /High-shrinkage yarn | Polyester | Round | 0.004 dtex /0.9 dtex. | 700 nm | 75T-112F (island component 30%:70%) /22T-24F | 5 | 7 | 216 × 113 | 98 | Woven fabric | PEDOT/PSS | 11.7 |
| Example 16 | Multifilament | Polyester | Round | 0.004 dtex | 700 nm | 75T-112F (island component 30%:70%) | 5 | 7 | 43 × 58 | 112 | Knitted fabric | PEDOT/PSS | 15.8 |
| Example 17 | Multifilament | Polyester | Round | 0.001 dtex | 300 nm | 100T-30F (island component 30%:70%) | 3 | 3.4 | 58 × 78 | 110 | Knitted fabric | PEDOT/PSS | 17.8 |
| Example 18 | Multifilament | Polyester | Round | 0.004 dtex | 200 nm | 120T-60F (island component 50%:50%) | 3 | 3.4 | 70 × 94 | 98 | Knitted fabric | PEDOT/PSS | 16.5 |
| Example 19 | Multifilament | Polyester | triangular | 0.004 dtex | 700 nm | 75T-112F (island component 30%:70%) | 3 | 3.4 | 43 × 58 | 115 | Knitted fabric | PEDOT/PSS | 16.3 |
| Example 20 | Multifilament | Polyester | Round | 0.07 dtex | 2700 nm | 66T-9F (island component 20% : 80%) | 6 | 9 | 114 × 118 | 61 | Knitted fabric | PEDOT/PSS | 9.8 |
| Example 21 | Multifilament | Polyester | Round | 0.15 dtex | 3800 nm | 0.15 dtex single-yarn fineness | 6 | 9 | - | 135 | Nonwoven fabric | PEDOT/PSS | 13.3 |

(continued)

| | Filament | Polymer | Cross-section | Fineness | Fiber diameter | Use of yarn | Variation of fiber diameter (CV% (A)) | Variation of modification ratio (CV% (B)) | Density (yarns /in) length x breadth | Areal weight (g/cm$^2$) | Fiber structure | Conductive polymer | Quantity of applied resin (g/cm$^2$) |
|---|---|---|---|---|---|---|---|---|---|---|---|---|---|
| Example 22 | Multifilament | Polyester | Round | 2.3 dtex. | 15000 nm | 84T-36F | 4 | 4.2 | 105 × 95 | 68 | Woven fabric | PEDOT/PSS | 12.2 |
| Example 23 | Multifilament | Polyester /polyurethane | Round | 2.3 dtex. | 15000 nm | 56T-24F /22T (PU) | 4 | 4.2 | 67 × 62 | 176 | Knitted fabric | PEDOT/PSS | 15.0 |
| Example 24 | Multifilament | Nylon | Round | 3.3 dtex | 36000 nm | 78T-24F | 3.5 | 3.7 | 32 × 40 | 88 | Knitted fabric | PEDOT/PSS | 15.6 |
| Example 25 | Multifilament /High-shrinkage yarn | Polyester | Round | 0.004 dtex /0.9 dtex. | 700 nm | 75T-112F (island component 30%:70%) /22T-24F | 5 | 7 | 58 × 78 | 118 | Knitted fabric | Polyaniline | 15.2 |
| Example 26 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | Polypyrrole | 14. 8 |
| Example 27 | Multifilament | Nylon | Round | 0.004 dtex | 700 nm | 75T-112F (island component 30%:70%) | 5 | 7 | 45 × 60 | 115 | Knitted fabric | PEDOT/PSS | 13.5 |
| Comparative Example 1 | R-PET film | | - | 0.10 mm thickness | - | - | - | - | - | 140 | Film | PEDOT/PSS | 15.5 |
| Comparative Example 2 | R-PET film | | - | 0.10mm thickness | - | - | - | - | - | 140 | Film | Hydrogel-based | 15.9 |

Table 2

| | moisture-permeable layer | dyeing | Chemical treatment | Physical treatment | Resistance ($\Omega$) | Resistance (washing) | Breathability (cc/cm2/ sec) | Bending Breath- resistance (mm) length $\times$ breadth |
|---|---|---|---|---|---|---|---|---|
| Example 1 | - | - | - | - | 57.7 | $1.1 \times 10^5$ | 150 | $15 \times 16$ |
| Example 2 | - | - | - | - | 63.1 | $0.42 \times 10^5$ | 180 | $22 \times 25$ |
| Example 3 | - | - | - | - | 36.5 | $1.4 \times 10^4$ | 0.521 | $47 \times 38$ |
| Example 4 | - | - | - | - | 60.3 | $2.8 \times 10^5$ | 140 | $12 \times 14$ |
| Example 5 | - | - | - | - | 35.2 | $1.8 \times 10^4$ | 130 | $10 \times 11$ |
| Example 6 | - | - | - | - | 25.5 | $2.5 \times 10^4$ | 126 | $10 \times 12$ |
| Example 7 | - | - | - | - | 64.5 | $2.4 \times 10^5$ | 135 | $15 \times 16$ |
| Example 8 | - | - | - | - | 29.3 | Equal to or more than $10^6$ | 43 | $39 \times 27$ |
| Example 9 | - | - | PU | Nap raising | 37.2 | $0.41 \times 10^4$ | 10.4 | $42 \times 43$ |
| Example 10 | - | - | - | - | 21.5 | Equal to or more than $10^6$ | Equal to or more than 600 | $49 \times 43$ |
| Example 11 | - | - | - | - | 16.5 | $0.32 \times 10^5$ | 250 | $25 \times 33$ |
| Example 12 | - | - | - | - | 22.1 | $0.98 \times 10^5$ | 401 | $37 \times 46$ |
| Example 13 | PU microporous | - | - | - | 15.3 | $0.22 \times 10^3$ | 0 | $32 \times 33$ |
| Example 14 | PU microporous | - | - | - | 19.3 | $0.28 \times 10^3$ | 0 | $38 \times 40$ |
| Example 15 | PU microporous | - | - | - | 30.3 | $0.40 \times 10^3$ | 0 | $69 \times 59$ |
| Example 16 | PU microporous | - | - | - | 16.8 | $1.4 \times 10^3$ | 0 | $25 \times 33$ |
| Example 17 | PU microporous | - | - | - | 14.8 | $2.3 \times 10^3$ | 0 | $23 \times 27$ |
| Example 18 | PU microporous | - | - | - | 14.5 | $0.82 \times 10^3$ | 0 | $24 \times 28$ |
| Example 19 | PU microporous | - | - | - | 15.1 | $0.43 \times 10^3$ | 0 | $29 \times 29$ |
| Example 20 | PU microporous | - | - | - | 38.3 | Equal to or more than $10^6$ | 0 | $76 \times 53$ |
| Example 21 | PU microporous | dyeing | PU | Nap raising | 38.1 | $0.57 \times 10^3$ | 0 | $42 \times 43$ |

| | moisture-permeable layer | dyeing | Chemical treatment | Physical treatment | Resistance ($\Omega$) | Resistance (washing) | Breathability (cc/cm2/ sec) | Bending Breath- resistance (mm) length $\times$ breadth |
|---|---|---|---|---|---|---|---|---|
| Example 22 | PU microporous | - | - | - | 21.3 | Equal to or more than $10^6$ | 0 | 69 $\times$ 57 |
| Example 23 | PU microporous | - | - | - | 16.6 | $0.4 \times 10^4$ | 0 | 17 $\times$ 23 |
| Example 24 | PU microporous | - | - | - | 16.1 | $0.29 \times 10^4$ | 0 | 36 $\times$ 52 |
| Example 25 | - | - | - | - | 43.2 | $6.8 \times 10^5$ | 160 | 18 $\times$ 19 |
| Example 26 | - | - | - | - | 50.8 | $7.2 \times 10^5$ | 165 | 20 $\times$ 21 |
| Example 27 | - | - | - | - | 40.3 | $1.4 \times 10^4$ | 138 | 25 $\times$ 33 |
| Comparative Example 1 | - | - | - | - | 14.8 | Equal to or more than $10^6$ | 0 | 8.7 |
| Comparative Example 2 | - | - | - | - | 790 | Equal to or more than $10^6$ | 0 | 9.2 |

EP 3 100 678 A1

Reference Signs List

| 100 | biosignal detecting garment |
| 101 | electrode materials |
| 102 | measurement device |
| 103 | wires |
| 104 | garment body |

## Claims

1. An electrode material comprising a fiber structure containing a conductive polymer, the conductive polymer being supported on surfaces of filaments constituting the fiber structure and/or in a gap between the filaments.

2. The electrode material according to claim 1, wherein
the fiber structure includes at least a multifilament yarn, and
a conductive substance is supported on surfaces of filaments constituting the multifilament yarn and/or in a gap between the filaments.

3. The electrode material according to claim 1 or 2, wherein a multifilament yarn constituting the fiber structure includes a filament of equal to or less than 0.2 dtex.

4. The electrode material according to any one of claims 1 to 3, wherein the conductive polymer is supported on the surfaces of the filaments constituting the fiber structure and/or in the gap between the filaments when the conductive polymer is dispersed with a binder in a solvent and the dispersion in which the conductive polymer is dispersed is applied to the fiber structure.

5. The electrode material according to any one of claims 1 to 4, wherein the conductive polymer is a mixture of poly(3,4-ethylenedioxythiophene) and polystyrenesulfonic acid.

6. The electrode material according to any one of claims 1 to 5, further comprising a resin layer layered on one face of the fiber structure containing the conductive substance.

7. The electrode material according to any one of claims 1 to 6, wherein the electrode material has a surface resistance of equal to or less than $1 \times 10^6 \, \Omega$ after 20 washing cycles in accordance with JIS L0217 (2012) 103 method.

8. The electrode material according to any one of claims 1 to 7, wherein the electrode material is layered in combination with an adhesive agent.

9. A device comprising the electrode material according to any one of claims 1 to 8, the electrode material being used as at least part of an electrode.

Figure 1

## INTERNATIONAL SEARCH REPORT

| International application No. |
| --- |
| PCT/JP2015/052226 |

**A. CLASSIFICATION OF SUBJECT MATTER**
*A61B5/0408*(2006.01)i, *A61B5/0478*(2006.01)i

According to International Patent Classification (IPC) or to both national classification and IPC

**B. FIELDS SEARCHED**

Minimum documentation searched (classification system followed by classification symbols)
A61B5/04-5/053, A61N1/00-1/44, D06M13/00-15/715

Documentation searched other than minimum documentation to the extent that such documents are included in the fields searched
Jitsuyo Shinan Koho        1922-1996   Jitsuyo Shinan Toroku Koho   1996-2015
Kokai Jitsuyo Shinan Koho  1971-2015   Toroku Jitsuyo Shinan Koho   1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2013/073673 A1 (Nippon Telegraph and Telephone Corp.),<br>23 May 2013 (23.05.2013),<br>claims 1, 4, 6 to 9; paragraphs [0005], [0013], [0015], [0020] to [0026], [0079], [0088] to [0104], [0110] to [0116], [0128], [0134] to [0136], [0192] to [0194], [0208] to [0246], [0298] to [0336]; fig. 1 to 26<br>& US 2014/0303470 A1   & EP 2767632 A1<br>& CN 103930612 A | 1-6,8-9<br>7 |
| Y | JP 2007-63704 A (Japan Exlan Co., Ltd.),<br>15 March 2007 (15.03.2007),<br>paragraphs [0027], [0029]<br>(Family: none) | 7 |

☒ Further documents are listed in the continuation of Box C.      ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 13 April 2015 (13.04.15) | 21 April 2015 (21.04.15) |

| Name and mailing address of the ISA/<br>Japan Patent Office<br>3-4-3,Kasumigaseki,Chiyoda-ku,<br>Tokyo 100-8915,Japan | Authorized officer |
| --- | --- |
| | Telephone No. |

Form PCT/ISA/210 (second sheet) (July 2009)

**INTERNATIONAL SEARCH REPORT**

| International application No. |
|---|
| PCT/JP2015/052226 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2007-37639 A (Transcutaneous Technologies Inc.), 15 February 2007 (15.02.2007), paragraphs [0017] to [0018], [0022], [0039], [0044], [0047], [0055], [0057], [0066], [0092]; fig. 1 to 2 (Family: none) | 1,6,8-9 |
| X | Microfilm of the specification and drawings annexed to the request of Japanese Utility Model Application No. 51220/1987(Laid-open No. 158309/1988) (Fukuda Denshi Co., Ltd.), 17 October 1988 (17.10.1988), specification, pages 6 to 8; fig. 1 to 5 (Family: none) | 1,4,6,8-9 |
| X | JP 5-212005 A (Japan Precision Instruments Inc.), 24 August 1993 (24.08.1993), paragraphs [0012] to [0015], [0021] to [0024]; fig. 1, 5 (Family: none) | 1,6,8-9 |
| X | JP 9-262299 A (Takiron Co., Ltd.), 07 October 1997 (07.10.1997), claim 1; paragraphs [0009] to [0022]; fig. 1 to 2 (Family: none) | 1,6,9 |
| A | JP 6-114019 A (Nihon Sanmo Dyeing Co., Ltd.), 26 April 1994 (26.04.1994), paragraph [0009] (Family: none) | 7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4860155 B
- JP 5135757 B
- JP 2007291562 A
- JP 4581467 B
- JP 2013216766 A
- JP 5472479 B
- JP 2013185283 A